(19) **Europäisches Patentamt / European Patent Office / Office européen des brevets**

(11) **EP 4 647 488 A1**

(12) **EUROPEAN PATENT APPLICATION**
published in accordance with Art. 153(4) EPC

(43) Date of publication:
**12.11.2025 Bulletin 2025/46**

(21) Application number: **24760634.6**

(22) Date of filing: **14.02.2024**

(51) International Patent Classification (IPC):
*C12N 1/12* (2006.01)   *C07K 14/405* (2006.01)
*C12R 1/89* (2006.01)

(86) International application number:
**PCT/KR2024/095110**

(87) International publication number:
**WO 2024/177446 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.02.2023 KR 20230022628**

(71) Applicant: **Daesang Corporation**
**Seoul 03130 (KR)**

(72) Inventors:
• **HWANG, Ji-Eun**
  **Seoul 03677 (KR)**
• **KIM, Bo-Ra**
  **Goyang-si Gyeonggi-do 10415 (KR)**
• **JEON, Jin-Young**
  **Seongnam-si Gyeonggi-do 13589 (KR)**

(74) Representative: **Turner, Craig Robert**
**Venner Shipley LLP**
**200 Aldersgate**
**London EC1A 4HD (GB)**

(54) **WHITE CHLORELLA PROTOTHECOIDES STRAIN HAVING HIGH CRUDE PROTEIN CONTENT AND METHOD FOR PRODUCING CHLORELLA BIOMASS USING SAME**

(57) The present invention provides a *Chlorella protothecoides* strain having a Hunter whiteness index (WI) of 70 or more and a crude protein content of 55 wt% or more; and a method for producing a *Chlorella* biomass comprising culturing the strain and recovering a biomass from the resulting culture solution.

**FIG. 2**

EP 4 647 488 A1

**Description**

**TECHNICAL FIELD**

The present invention relates to a white *Chlorella protothecoides* strain having a high crude protein content and a method for producing a *Chlorella* biomass from a culture solution of the strain.

**BACKGROUND ART**

Chlorella, which is one of the freshwater green microalgaes, contains nutrients such as carbohydrates, proteins, fats, as well as chlorophyll (i.e., a green pigment referred to as chlorophyll), vitamins, minerals, and the like, which are beneficial to the human body. Thus, chlorella is known as a raw material for foods with excellent nutritional value and has been selected even as a space food. In addition, chlorella, which is widely used as a raw material for health functional foods, not only has a high antioxidant effect, but can also help improve skin health, immune function, and blood cholesterol. Chlorella is cultured by autotrophic culture, heterotrophic culture, or a combination thereof and may be cultured in a medium containing a carbon source, a nitrogen source, a trace metal, a trace element, and the like. Green chlorella containing chlorophyll is unstable to pH and/or heat and thus has a characteristic that is easily changed into brown color.

*Chlorella*-derived crude proteins, i.e., the crude proteins contained in *Chlorella* strains, comprise pure proteins, nucleic acids, amide compounds, urea, and the like. The present inventors have developed a *Chlorella* strain having high crude protein productivity [e.g., *Chlorella protothecoides* DS-NCRC7 (KCTC 18633P)] and a method for producing *Chlorella*-derived crude proteins using the same (Korean Patent No. 10-2026681).

Meanwhile, International Patent Publication No. WO 2010/120923 discloses a microalgal food composition comprising microalgal biomass. The microalgal biomass is a biomass of the white color mutant selected from the *Chlorella protothecoides* stains through treatment with N-methyl-N'-nitro-N-nitrosoguanidine (NTG) [i.e., *Chlorella protothecoides* 33-55 (PTA-10396)]. However, the cell body (biomass) of *Chlorella protothecoides* 33-55 (PTA-10396) disclosed in International Patent Publication No. WO 2010/120923 has a high lipid content (approximately 68%) and thus a relatively low crude protein content (e.g., up to 32% even if the amounts of carbohydrates, moisture, and ash are assumed to be 0%).

Accordingly, there is a need in the art to discover a *Chlorella protothecoides* strain providing a biomass capable of solving the problem of color development caused when added to foods and the like, as well as having a high crude protein content within the biomass.

**DISCLOSURE**

**Technical Problem**

The present inventors have carried out various studies to develop a white *Chlorella protothecoides* strain having high crude protein productivity. Especially, the present inventors obtained various mutants through N-methyl-N'-nitro-N-nitrosoguanidine (NTG) treatment on *Chlorella protothecoides* DS-NCRC7 (KCTC 18633P), which had been developed by the present inventors, and evaluated whiteness and crude protein productivity of the obtained mutants. As a result, the present inventors have found that the selected specific strain not only exhibits high crude protein productivity but also provides a biomass having white color.

Therefore, it is an object of the present invention to provide the novel *Chlorella protothecoides* strain selected as in the above.

It is another object of the present invention to provide a method for producing a *Chlorella* biomass comprising culturing the novel *Chlorella protothecoides* strain and recovering a biomass therefrom.

**Technical Solution**

In accordance with an aspect of the present invention, there is provided a *Chlorella protothecoides* strain, having a whiteness index (WI) of 70 or more as calculated from the following formula and a crude protein content of 55 wt% or more in a biomass thereof.

$$\text{WI} = 100 - \sqrt{(100 - L)^2 + a^2 + b^2}$$

wherein, L, a, and b are Hunter L, a, and b values.

In the *Chlorella protothecoides* strain according to the present invention, the crude protein content in the biomass may is 55 to 80 wt%. A chlorophyll content may be 3.0 or less mg/g dry cell wight, e.g., 0.1 to 3.0 mg/g dry cell wight. In an embodiment, the strain may be *Chlorella protothecoides* DS-NCRC7W (KCTC 15163BP).

In accordance with another aspect of the present invention, there is provided a method for producing a *Chlorella* biomass, comprising culturing said *Chlorella protothecoides* strain; and recovering a biomass from the resulting culture solution.

In the method of the present invention, the strain may be *Chlorella protothecoides* DS-NCRC7W (KCTC 15163BP). The culturing may be carried out in an aqueous medium having a pH of 6.5 to 7.5, containing 2-15 g/L of $MgSO_4$, 0.5-5 g/L of $KH_2PO_4$, 0.0001-0.1 g/L of $ZnSO_4$, 0.0001-0.01 g/L of $CuSO_4$, 0.001-0.1 g/L of $CaCl_2$, and 0.001-0.1 g/L of $FeSO_4$.

**ADVANTAGEOUS EFFECTS**

The *Chlorella protothecoides* strain obtained according to the present invention, particularly *Chlorella protothecoides* DS-NCRC7W (KCTC 15163BP), can not only exhibit high crude protein productivity but also provide a biomass having white color. Therefore, the strain and the *Chlorella* biomass obtained therefrom can effectively solve the problem of color development caused when added to foods and the like.

**DESCRIPTION OF DRAWINGS**

FIG. 1 shows the appearances of the culture solutions obtained by culturing under shaking the *Chlorella protothecoides* mutant strains obtained according to the present invention.

FIG. 2 shows the appearance of the powder obtained by spray drying a culture concentrate of the novel *Chlorella protothecoides* strain obtained according to the present invention.

FIG. 3 shows the results obtained by measuring the appearance of the powder obtained by spray drying a culture concentrate of the novel *Chlorella protothecoides* strain obtained according to the present invention, with a colorimeter.

**BEST MODE**

As used herein, Whiteness Index (WI) refers to the degree of whiteness calculated according to a known method (e.g., Chin-Lin Hsu et al., Food Chemistry 83 (2003) 85-92). Specifically, the whiteness index (WI) is a value calculated by the following formula from the Hunter values obtained by measuring from three spots of the sample with a colorimeter (colour differential meter), i.e., Hunter L, a, and b values (average values for five samples each).

<Formula 1>

$$ \mathrm{WI} = 100 - \sqrt{(100 - L)^2 + a^2 + b^2} $$

wherein, L, a, and b are Hunter L, a, and b values.

The present invention provides a *Chlorella protothecoides* strain, having a whiteness index (WI) of 70 or more as calculated from the above formula and a crude protein content of 55 wt% or more in a biomass thereof.

The present inventors carried out random mutation by treating the known *Chlorella protothecoides* DS-NCRC7 (KCTC 18633P) twice with N-methyl-N'-nitro-N-nitrosoguanidine (NTG) and selected a mutant strain exhibiting high cell mass and crude protein content as well as exhibiting white color. And, the present inventors confirmed that the selected mutant strain could be effectively cultured through fed-batch cultivation in a 5 L jar fermenter. The selected mutant strain was named *Chlorella protothecoides* DS-NCRC7W and deposited at the Korean Collection for Type Culture (KCTC) of Korea Research Institute of Bioscience and Biotechnology on October 31, 2022, and was assigned the accession number KCTC 15163BP.

In the *Chlorella protothecoides* strain according to the present invention, the crude protein content in the biomass may is preferably 55 to 80 wt%. And, *the Chlorella protothecoides* strain according to the present invention has a low chlorophyll content. For example, the chlorophyll content in *the Chlorella protothecoides* strain according to the present invention may be 3.0 or less mg/g dry cell wight (DCW), e.g., 0.1 to 3.0 mg/g dry cell wight. In an embodiment, the strain may be *Chlorella protothecoides* DS-NCRC7W (KCTC 15163BP).

The present invention also provides a method for producing a *Chlorella* biomass, comprising culturing said novel *Chlorella protothecoides* strain; and recovering a biomass from the resulting culture solution.

The culturing may be carried out according to conventional culture methods of a *Chlorella* strain (e.g., Republic of Korea Patent No. 10-2026681). For example, the culturing may be carried out in a medium containing glucose as a sugar source, phosphates ($KH_2PO_4$, $K_2HPO_4$, etc.), ammonium salts (($NH_4)_2SO_4$, etc.), calcium salts ($CaCl_2$, etc.), metal salts ($MgSO_4$, $ZnSO_4$, $CuSO_4$, $FeSO_4$, etc.), etc., in water (e.g., purified water, etc.). In an embodiment, the culturing may be carried out in an aqueous medium having a pH of 6.5 to 7.5, containing 2-15 g/L of $MgSO_4$, 0.5-5 g/L of $KH_2PO_4$, 0.0001-0.1 g/L of $ZnSO_4$, 0.0001-0.01 g/L of $CuSO_4$, 0.001-0.1 g/L of $CaCl_2$, and 0.001-0.1 g/L of $FeSO_4$. And, if necessary, the medium may further include an antifoaming agent or the like. The culturing in the medium described above may be carried out according to conventional culture methods, for example, according to fed-batch culture in a sterile tank, under 0.3-1.0 vvm of air and 200-500 rpm of agitation.

The recovering a biomass may be carried out by recovering a biomass, e.g., through centrifuging the culture solution. The obtained biomass may be concentrated to an appropriate concentration through a plurality of concentration and washing steps as needed. The *Chlorella* biomass may be obtained in a powder form through conventional methods, for example, spray drying or drum drying.

The present invention will be described in further detail with reference to the following examples. These examples are for illustrative purposes only and are not intended to limit the scope of the present invention.

**Example 1: Selection of mutant strains through random mutation**

(1) Primary selection of mutant strains

For performing random mutation, *Chlorella protothecoides* DS-NCRC7 (KCTC 18633P) was treated with N-methyl-N'-nitro-N-nitrosoguanidine (NTG). Specifically, *Chlorella protothecoides* DS-NCRC7 (KCTC 18633P) was treated with NTG for about 30 minutes, spread on the plates containing a medium (glucose 15 g/L, $KH_2PO_4$ 1 g/L, $ZnSO_4$ 0.001 g/L, and agar 20 g/L), and then cultured for about 15 days. After about 60 strains exhibiting a white color were selected from the plates, the strains were pooled in distilled water and then two mutants exhibiting a white color were selected.

(2) Secondary selection of mutant strains

The two mutant strains selected in (1) above were treated with NTG for about 30 minutes, spread on plates containing a medium (glucose 15 g/L, $MgSO_4$ 2 g/L, $KH_2PO_4$ 1 g/L, $ZnSO_4$ 0.001 g/L, $FeSO_4$ 0.03 g/L, and agar 20 g/L) in the same manner as in (1), and then cultured for about 15 days. After about 40 mutants exhibiting a white color were obtained from the plates, the mutants were pooled in distilled water and then two mutants exhibiting a white color were selected.

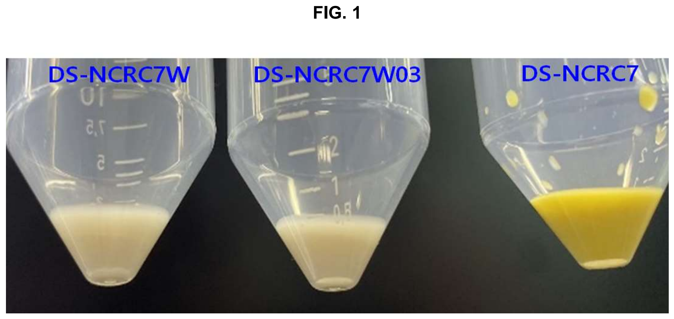

In order to confirm the protein contents and growths of the secondary-selected mutants, a medium [an aqueous solution (pH 7.0) containing glucose 10 g/L, $MgSO_4$ 0.3 g/L, $KH_2PO_4$ 0.7 g/L, $ZnSO_4$ 0.001 g/L, and $FeSO_4$ 0.03 g/L] was added to a 500 mL baffled flask and then cultured under shaking. The above shaking culture was carried out for 48 hours under the conditions of a starting liquid volume of 100 ml, a culture temperature of 28°C, and an agitation speed of 140 rpm. After performing the shaking culture for 48 hours as described above, the cell mass and crude protein content from each culture flask were measured. The cell mass was obtained by centrifuging the culture solution to remove the medium, washing the pellet twice with distilled water, drying the resulting cell suspension at 105°C for 24 hours, and then measuring the weight of the resultant. The crude protein content was obtained by centrifuging the culture solution to remove the medium, washing the pellet twice with distilled water, adding sulfuric acid ($H_2SO_4$) and a decomposition accelerator, Kjeltabs S-3.4 (manufactured by Foss) thereto for heat-decomposition, and then measuring with the Kjeldahl nitrogen quantitative assay method. The results are shown in Table 1 below. And, the appearances of the culture solutions obtained through the shaking culture are as shown in Figure 1.

Table 1

| Mutant | Cell mass (g/L) | Crude protein (%) |
|---|---|---|
| DS-NCRC7W | 4.8 | 67.21 |
| DS-NCRC7W03 | 4.4 | 66.04 |

As can be seen from the results of Table 1 above, it can be confirmed that DS-NCRC7W exhibits high cell mass and crude protein content. The above-selected mutant strain, DS-NCRC7W, was named *Chlorella protothecoides* DS-NCRC7W and deposited at the Korean Collection for Type Culture (KCTC) of Korea Research Institute of Bioscience and Biotechnology on October 31, 2022, and was assigned the accession number KCTC 15163BP.

**Example 2: 5L JF culture of *Chlorella protothecoides* DS-NCRC7W**

A medium [an aqueous solution (pH 7.2) containing glucose 40 g/L, MgSO$_4$ 3 g/L, KH$_2$PO$_4$ 2 g/L, ZnSO$_4$ 0.05 g/L, CuSO$_4$ 0.0005 g/L, CaCl$_2$ 0.04 g/L, and FeSO$_4$ 0.01 g/L] was added to a 5 L jar fermenter and the DS-NCRC7W strain [i.e., *Chlorella protothecoides* DS-NCRC7W (KCTC 15163BP)] was cultured under conditions of an agitation speed of 500 rpm and aeration of 1.6 vvm for approximately 3 days. After completion of the culture, a certain amount was taken, and the dry cell weight (DCW) and crude protein content were measured. The cell mass was obtained by centrifuging the culture solution to remove the medium, washing the pellet twice with distilled water, drying the resulting cell suspension at 105°C for 24 hours, and then measuring the weight of the resultant. The crude protein content was obtained by centrifuging the culture solution to remove the medium, washing the pellet twice with distilled water, adding sulfuric acid (H$_2$SO$_4$) and a decomposition accelerator, Kjeltabs S-3.4 (manufactured by Foss) thereto for heat-decomposition, and then measuring with the Kjeldahl nitrogen quantitative assay method. The results are shown in Table 2 below.

Table 2

| Mutant | Cell mass (g/L) | Crude protein (%) |
|---|---|---|
| DS-NCRC7W | 43.4 | 60.12 |

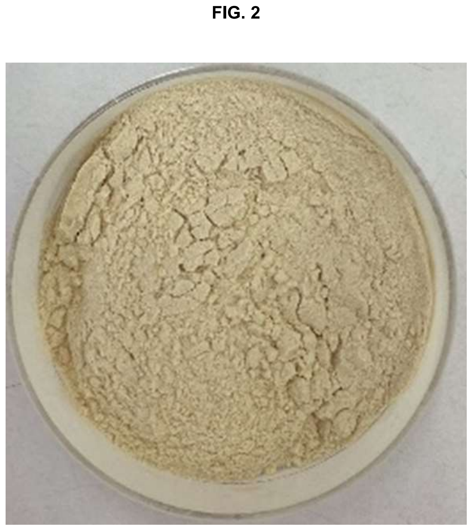

In addition, the culture solution was centrifuged to collect the supernatant, which was concentrated approximately twice to obtain a culture concentrate. 1 L of the obtained culture concentrate was spray-dried to obtain a powder, and the appearance of the obtained powder is shown in FIG. 2.

**Example 3: Measurement of chlorophyll content and colorimetric measurement**

The DS-NCRC7W strain obtained according to the present invention [i.e., *Chlorella protothecoides* DS-NCRC7W (KCTC 15163BP)], *Chlorella protothecoides* DS-NCRC7 (KCTC 18633P), and *Chlorella vulgaris* (dsv77) were cultured in a 5 L jar fermenter, respectively. Each powder obtained therefrom was used as a sample. Each total chlorophyll content was measured through the test method according to the standards and regulations of health functional food of Ministry of Food and Drug Safety (MFDS) [i.e., Total chlorophyll measurement protocol (First method)]. Specifically, distilled water was added to each powder, allowed to stand for 30 minutes, placed in a centrifugal tube, mixed with an alkaline pyridine solution, allowed to stand for 15 minutes in a 60°C water bath, and then centrifuged to obtain a supernatant. The total chlorophyll contents therein were quantified by measuring the absorbance at wavelengths of 419 nm and 454 nm against an alkaline pyridine solution. The results are shown in Table 3 below.

Table 3

| Strain | Total chlorophyll (mg/g) |
|---|---|
| DS-NCRC7W [*Chlorella protothecoides* DS-NCRC7W (KCTC 15163BP)] | 0.72 |
| DS-NCRC7 [*Chlorella protothecoides* DS-NCRC7 (KCTC 18633P)] | 0.21 |
| *Chlorella vulgaris* (dsv77) | 28.64 |

In addition, Hunter L, a, and b values (mean and SD) were obtained for each powder (five samples per powder) using a colorimeter (manufactured by Konica Minolta), and the whiteness indexes (WI) were calculated from these values using the following formula. The results are shown in Table 4.

<Formula 1>

$$WI = 100 - \sqrt{(100 - L)^2 + a^2 + b^2}$$

wherein, L, a, and b are Hunter L, a, and b values.

Table 4

| Sample | Value | Mean | SD | Whiteness Index (WI) | Appearance |
|---|---|---|---|---|---|
| DS-NCRC7W [*Chlorella protothecoides* DS-NCRC7W (KCTC 15163BP)] | L | 87.62 | 0.02 | 79 | FIG. 3 (A) |
| | a | 0.54 | 0.02 | | |
| | b | 16.72 | 0.09 | | |
| DS-NCRC7 [*Chlorella protothecoides* DS-NCRC7 (KCTC 18633P)] | L | 75.76 | 0.09 | 31 | FIG. 3 (B) |
| | a | 2.88 | 0.08 | | |
| | b | 64.18 | 0.23 | | |
| *Chlorella vulgaris* (dsv77) | L | 21.25 | 0.14 | 21 | FIG. 3 (C) |
| | a | -3.75 | 0.13 | | |
| | b | 6.06 | 0.17 | | |

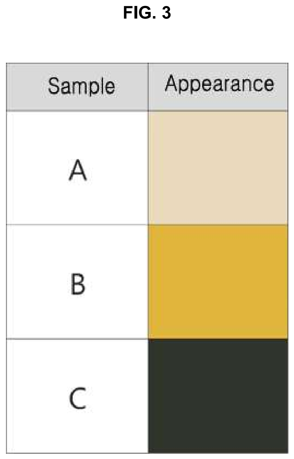

From the results of Table 3 and Table 4 (Fig. 3), it can be confirmed that the DS-NCRC7W strain selected according to the present invention [i.e., *Chlorella protothecoides* DS-NCRC7W (KCTC 15163BP)] has a chlorophyll content of 3.0 mg/g or less dry cell weight and exhibits a white color.

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: **Daesang Corporation**

Daesang Corporation

178 Magokjungang-ro, Gangseo-gu, Seoul

Republic of Korea

| I. IDENTIFICATION OF THE MICROORGANISM | |
|---|---|
| Identification reference given by the DEPOSITOR:<br><br>*Chlorella protothecoides* **DS-NCRC7W** | Accession number given by the INTERNATIONAL DEPOSITARY AUTHORITY:<br><br>**KCTC 15163BP** |

| II. SCIENTIFIC DESCRIPTION AND/OR PROPOSED TAXONOMIC DESIGNATION |
|---|
| The microorganism identified under I above was accompanied by:<br><br>  [   ] a scientific description<br><br>  [   ] a proposed taxonomic designation<br><br>(Mark with a cross where applicable) |

| III. RECEIPT AND ACCEPTANCE |
|---|
| This International Depositary Authority accepts the microorganism identified under I above, which was received by it on **October 31, 2022.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures**<br><br>Address: Korea Research Institute of<br>       Bioscience and Biotechnology (KRIBB)<br>       181, Ipsin-gil, Jeongeup-si, Jeolllabuk-do 56212<br>       Republic of Korea | Signature(s) of person(s) having the power to represent the International Depositary Authority or of authorized official(s):<br><br>*Song Gun Kim*<br><br>KIM, Song-Gun, Director<br>Date: **November 30, 2022** |

Form BP/4 (KCTC Form 17)                                                                                                            sole page

(Reissue)

## Claims

**1.** A *Chlorella protothecoides* strain, having a whiteness index (WI) of 70 or more as calculated from the following formula and a crude protein content of 55 wt% or more in a biomass thereof.

$$\text{WI} = 100 - \sqrt{(100 - L)^2 + a^2 + b^2}$$

wherein, L, a, and b are Hunter L, a, and b values.

2. The *Chlorella protothecoides* strain according to claim 1, wherein a crude protein content in the biomass is 55 to 80 wt%.

3. The *Chlorella protothecoides* strain according to claim 1, wherein a chlorophyll content is 3.0 or less mg/g dry cell wight.

4. The *Chlorella protothecoides* strain according to claim 3, wherein a chlorophyll content is 0.1 to 3.0 mg/g dry cell wight.

5. The *Chlorella protothecoides* strain according to any one of claims 1 to 4, wherein the strain is *Chlorella protothecoides* DS-NCRC7W (KCTC 15163BP).

6. A method for producing a *Chlorella* biomass, comprising culturing a *Chlorella protothecoides* strain according to any one of claims 1 to 4; and recovering a biomass from the resulting culture solution.

7. The method according to claim 6, wherein the strain is *Chlorella protothecoides* DS-NCRC7W (KCTC 15163BP).

8. The method according to claim 6, wherein the culturing is carried out in an aqueous medium having a pH of 6.5 to 7.5, containing 2-15 g/L of $MgSO_4$, 0.5-5 g/L of $KH_2PO_4$, 0.0001-0.1 g/L of $ZnSO_4$, 0.0001-0.01 g/L of $CuSO_4$, 0.001-0.1 g/L of $CaCl_2$, and 0.001-0.1 g/L of $FeSO_4$.

**FIG. 1**

FIG. 2

**FIG. 3**

| Sample | Appearance |
|--------|------------|
| A | |
| B | |
| C | |

## INTERNATIONAL SEARCH REPORT

| International application No. |
| --- |
| **PCT/KR2024/095110** |

| **A.** | **CLASSIFICATION OF SUBJECT MATTER** |
| --- | --- |

**C12N 1/12**(2006.01)i; **C07K 14/405**(2006.01)i; C12R 1/89(2006.01)n

According to International Patent Classification (IPC) or to both national classification and IPC

| **B.** | **FIELDS SEARCHED** |
| --- | --- |

Minimum documentation searched (classification system followed by classification symbols)

C12N 1/12(2006.01); A23J 3/20(2006.01); A23L 1/10(2006.01); A23L 1/32(2006.01); A23L 17/60(2016.01); A23L 29/00(2016.01); C07K 14/405(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 헌터 백색도(Hunter Whiteness Index), 조단백질(crude protein), 클로렐라 프로토데코이데스(Chlorella prototothecoides), DS-NCRC7W, 배양(culture)

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| DX | US 2010-0297292 A1 (BROOKS, Geoffrey et al.) 25 November 2010 (2010-11-25) See paragraphs [0012]-[0014], [0102], [0108]-[0109], [0151]-[0152] and [0385]. | 1-4,6,8 |
| DA | | 5,7 |
| DA | KR 10-2026681 B1 (DAESANG CORPORATION) 30 September 2019 (2019-09-30) See entire document. | 1-8 |
| A | KR 10-2018-0009742 A (ROQUETTE FRERES) 29 January 2018 (2018-01-29) See entire document. | 1-8 |
| A | KR 10-2015-0110752 A (SOLAZYME ROQUETTE NUTRITIONALS, LLC) 02 October 2015 (2015-10-02) See entire document. | 1-8 |

| ✓ Further documents are listed in the continuation of Box C. | ✓ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **16 May 2024** | **17 May 2024** |

| Name and mailing address of the ISA/KR | Authorized officer |
| --- | --- |
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2024/095110** |

### C.    DOCUMENTS CONSIDERED TO BE RELEVANT

| Category* | Citation of document, with indication, where appropriate, of the relevant passages | Relevant to claim No. |
| --- | --- | --- |
| A | GROSSMANN, L. et al. Effect of precipitation, lyophilization, and organic solvent extraction on preparation of protein-rich powders from the microalgae Chlorella protothecoides. Algal Research. 2018, vol. 29, pp. 266-276.<br>    See entire document. | 1-8 |

Form PCT/ISA/210 (second sheet) (July 2022)

## INTERNATIONAL SEARCH REPORT
### Information on patent family members

International application No.

**PCT/KR2024/095110**

| Patent document cited in search report | | | Publication date (day/month/year) | Patent family member(s) | | | Publication date (day/month/year) |
|---|---|---|---|---|---|---|---|
| US | 2010-0297292 | A1 | 25 November 2010 | CN | 102057048 | A | 11 May 2011 |
| | | | | CN | 102057048 | B | 21 January 2015 |
| | | | | CN | 102271525 | A | 07 December 2011 |
| | | | | CN | 102271525 | B | 07 January 2015 |
| | | | | CN | 102300996 | A | 28 December 2011 |
| | | | | CN | 102300996 | B | 01 June 2018 |
| | | | | CN | 102575271 | A | 11 July 2012 |
| | | | | CN | 102712858 | A | 03 October 2012 |
| | | | | CN | 102712858 | B | 12 August 2015 |
| | | | | CN | 102946738 | A | 27 February 2013 |
| | | | | CN | 102946738 | B | 01 April 2015 |
| | | | | CN | 104561150 | A | 29 April 2015 |
| | | | | CN | 104770424 | A | 15 July 2015 |
| | | | | CN | 104783175 | A | 22 July 2015 |
| | | | | CN | 104783175 | B | 22 August 2017 |
| | | | | CN | 106367198 | A | 01 February 2017 |
| | | | | CN | 107034142 | A | 11 August 2017 |
| | | | | EP | 2265724 | A2 | 29 December 2010 |
| | | | | EP | 2265724 | A4 | 23 January 2013 |
| | | | | EP | 2339925 | A2 | 06 July 2011 |
| | | | | EP | 2339925 | A4 | 29 April 2015 |
| | | | | EP | 2339925 | B1 | 21 September 2022 |
| | | | | EP | 2370554 | A2 | 05 October 2011 |
| | | | | EP | 2370554 | A4 | 18 June 2014 |
| | | | | EP | 2370554 | B1 | 15 May 2019 |
| | | | | EP | 2370555 | A2 | 05 October 2011 |
| | | | | EP | 2370555 | A4 | 15 October 2014 |
| | | | | EP | 2370555 | B1 | 20 April 2016 |
| | | | | EP | 2411002 | A1 | 01 February 2012 |
| | | | | EP | 2411002 | A4 | 20 August 2014 |
| | | | | EP | 2411002 | B1 | 02 January 2019 |
| | | | | EP | 2418959 | A1 | 22 February 2012 |
| | | | | EP | 2418959 | A4 | 27 May 2015 |
| | | | | EP | 2418959 | B1 | 24 July 2019 |
| | | | | EP | 2419520 | A2 | 22 February 2012 |
| | | | | EP | 2419520 | A4 | 10 August 2016 |
| | | | | EP | 2419520 | B1 | 26 June 2019 |
| | | | | EP | 3098321 | A2 | 30 November 2016 |
| | | | | EP | 3098321 | A3 | 11 January 2017 |
| | | | | EP | 3098321 | B1 | 19 December 2018 |
| | | | | EP | 3517622 | A1 | 31 July 2019 |
| | | | | EP | 3622828 | A1 | 18 March 2020 |
| | | | | EP | 3622828 | B1 | 16 November 2022 |
| | | | | JP | 2011-519549 | A | 14 July 2011 |
| | | | | JP | 2012-505656 | A | 08 March 2012 |
| | | | | JP | 2012-510275 | A | 10 May 2012 |
| | | | | JP | 2012-513743 | A | 21 June 2012 |
| | | | | JP | 2012-523843 | A | 11 October 2012 |
| | | | | JP | 2014-061009 | A | 10 April 2014 |
| | | | | JP | 2014-128291 | A | 10 July 2014 |

Form PCT/ISA/210 (patent family annex) (July 2022)

14

## INTERNATIONAL SEARCH REPORT
### Information on patent family members

International application No.

**PCT/KR2024/095110**

| Patent document cited in search report | Publication date (day/month/year) | Patent family member(s) | | | Publication date (day/month/year) |
|---|---|---|---|---|---|
| | | JP | 2014-138620 | A | 31 July 2014 |
| | | JP | 2015-033388 | A | 19 February 2015 |
| | | JP | 2015-216928 | A | 07 December 2015 |
| | | JP | 2016-116535 | A | 30 June 2016 |
| | | JP | 2016-182144 | A | 20 October 2016 |
| | | JP | 2016-198117 | A | 01 December 2016 |
| | | JP | 2017-140019 | A | 17 August 2017 |
| | | JP | 2018-075040 | A | 17 May 2018 |
| | | JP | 2018-126167 | A | 16 August 2018 |
| | | JP | 2020-072663 | A | 14 May 2020 |
| | | JP | 5636039 | B2 | 03 December 2014 |
| | | JP | 5731982 | B2 | 10 June 2015 |
| | | JP | 5859311 | B2 | 10 February 2016 |
| | | JP | 6066463 | B2 | 25 January 2017 |
| | | JP | 6109475 | B2 | 05 April 2017 |
| | | JP | 6386727 | B2 | 05 September 2018 |
| | | KR | 10-1607235 | B1 | 29 March 2016 |
| | | KR | 10-1763878 | B1 | 01 August 2017 |
| | | KR | 10-1769121 | B1 | 17 August 2017 |
| | | KR | 10-1780799 | B1 | 22 September 2017 |
| | | KR | 10-1888973 | B1 | 16 August 2018 |
| | | KR | 10-1899933 | B1 | 19 September 2018 |
| | | KR | 10-2001129 | B1 | 17 July 2019 |
| | | KR | 10-2010-0131009 | A | 14 December 2010 |
| | | KR | 10-2011-0091567 | A | 11 August 2011 |
| | | KR | 10-2011-0096555 | A | 30 August 2011 |
| | | KR | 10-2011-0138407 | A | 27 December 2011 |
| | | KR | 10-2017-0091170 | A | 08 August 2017 |
| | | KR | 10-2017-0096218 | A | 23 August 2017 |
| | | KR | 10-2017-0107595 | A | 25 September 2017 |
| | | US | 10260076 | B2 | 16 April 2019 |
| | | US | 10278912 | B2 | 07 May 2019 |
| | | US | 11542456 | B2 | 03 January 2023 |
| | | US | 2009-0305942 | A1 | 10 December 2009 |
| | | US | 2010-0151112 | A1 | 17 June 2010 |
| | | US | 2010-0151535 | A1 | 17 June 2010 |
| | | US | 2010-0151538 | A1 | 17 June 2010 |
| | | US | 2010-0151539 | A1 | 17 June 2010 |
| | | US | 2010-0151567 | A1 | 17 June 2010 |
| | | US | 2010-0170144 | A1 | 08 July 2010 |
| | | US | 2010-0239712 | A1 | 23 September 2010 |
| | | US | 2010-0297295 | A1 | 25 November 2010 |
| | | US | 2010-0297296 | A1 | 25 November 2010 |
| | | US | 2010-0297323 | A1 | 25 November 2010 |
| | | US | 2010-0297325 | A1 | 25 November 2010 |
| | | US | 2010-0297331 | A1 | 25 November 2010 |
| | | US | 2010-0303957 | A1 | 02 December 2010 |
| | | US | 2010-0303961 | A1 | 02 December 2010 |
| | | US | 2010-0303989 | A1 | 02 December 2010 |
| | | US | 2010-0303990 | A1 | 02 December 2010 |

Form PCT/ISA/210 (patent family annex) (July 2022)

## INTERNATIONAL SEARCH REPORT
### Information on patent family members

International application No.

**PCT/KR2024/095110**

| Patent document cited in search report | Publication date (day/month/year) | Patent family member(s) | | Publication date (day/month/year) |
|---|---|---|---|---|
| | | US | 2011-0165634 A1 | 07 July 2011 |
| | | US | 2011-0203168 A1 | 25 August 2011 |
| | | US | 2011-0250658 A1 | 13 October 2011 |
| | | US | 2012-0128851 A1 | 24 May 2012 |
| | | US | 2012-0130099 A1 | 24 May 2012 |
| | | US | 2012-0149075 A1 | 14 June 2012 |
| | | US | 2012-0202768 A1 | 09 August 2012 |
| | | US | 2012-0277453 A1 | 01 November 2012 |
| | | US | 2012-0283460 A1 | 08 November 2012 |
| | | US | 2013-0005005 A1 | 03 January 2013 |
| | | US | 2013-0006006 A1 | 03 January 2013 |
| | | US | 2013-0078709 A1 | 28 March 2013 |
| | | US | 2013-0089916 A1 | 11 April 2013 |
| | | US | 2013-0122180 A1 | 16 May 2013 |
| | | US | 2013-0165677 A1 | 27 June 2013 |
| | | US | 2013-0273621 A1 | 17 October 2013 |
| | | US | 2013-0295268 A1 | 07 November 2013 |
| | | US | 2013-0296591 A1 | 07 November 2013 |
| | | US | 2014-0249342 A1 | 04 September 2014 |
| | | US | 2014-0256024 A1 | 11 September 2014 |
| | | US | 2014-0305031 A1 | 16 October 2014 |
| | | US | 2014-0336100 A1 | 13 November 2014 |
| | | US | 2015-0150776 A1 | 04 June 2015 |
| | | US | 2015-0218604 A1 | 06 August 2015 |
| | | US | 2016-0324167 A1 | 10 November 2016 |
| | | US | 2017-0314048 A1 | 02 November 2017 |
| | | US | 2018-0139994 A1 | 24 May 2018 |
| | | US | 2018-0216027 A1 | 02 August 2018 |
| | | US | 2019-0254291 A1 | 22 August 2019 |
| | | US | 2019-0254292 A1 | 22 August 2019 |
| | | US | 2021-0244064 A1 | 12 August 2021 |
| | | US | 7883882 B2 | 08 February 2011 |
| | | US | 7935515 B2 | 03 May 2011 |
| | | US | 8119583 B2 | 21 February 2012 |
| | | US | 8187860 B2 | 29 May 2012 |
| | | US | 8222010 B2 | 17 July 2012 |
| | | US | 8268610 B2 | 18 September 2012 |
| | | US | 8278261 B2 | 02 October 2012 |
| | | US | 8435767 B2 | 07 May 2013 |
| | | US | 8450083 B2 | 28 May 2013 |
| | | US | 8674180 B2 | 18 March 2014 |
| | | US | 8697427 B2 | 15 April 2014 |
| | | US | 8772575 B2 | 08 July 2014 |
| | | US | 8822176 B2 | 02 September 2014 |
| | | US | 8822177 B2 | 02 September 2014 |
| | | US | 8927522 B2 | 06 January 2015 |
| | | US | 8951777 B2 | 10 February 2015 |
| | | US | 9062294 B2 | 23 June 2015 |
| | | US | 9353389 B2 | 31 May 2016 |
| | | US | 9464304 B2 | 11 October 2016 |

Form PCT/ISA/210 (patent family annex) (July 2022)

**INTERNATIONAL SEARCH REPORT**
Information on patent family members

International application No.

**PCT/KR2024/095110**

| Patent document cited in search report | | | Publication date (day/month/year) | Patent family member(s) | | | Publication date (day/month/year) |
|---|---|---|---|---|---|---|---|
| | | | | US | 9593351 | B2 | 14 March 2017 |
| | | | | US | 9896642 | B2 | 20 February 2018 |
| | | | | WO | 2009-126843 | A2 | 15 October 2009 |
| | | | | WO | 2009-126843 | A3 | 30 December 2009 |
| | | | | WO | 2010-045368 | A2 | 22 April 2010 |
| | | | | WO | 2010-045368 | A3 | 24 June 2010 |
| | | | | WO | 2010-063031 | A2 | 03 June 2010 |
| | | | | WO | 2010-063031 | A3 | 22 November 2012 |
| | | | | WO | 2010-063032 | A2 | 03 June 2010 |
| | | | | WO | 2010-063032 | A3 | 14 October 2010 |
| | | | | WO | 2010-111710 | A1 | 30 September 2010 |
| | | | | WO | 2010-120923 | A1 | 21 October 2010 |
| | | | | WO | 2010-120939 | A2 | 21 October 2010 |
| | | | | WO | 2010-120939 | A3 | 09 December 2010 |
| KR | 10-2026681 | B1 | 30 September 2019 | KR | 10-2019-0095675 | A | 16 August 2019 |
| KR | 10-2018-0009742 | A | 29 January 2018 | CN | 107635411 | A | 26 January 2018 |
| | | | | EP | 3298126 | A1 | 28 March 2018 |
| | | | | EP | 3298126 | B1 | 08 January 2020 |
| | | | | JP | 2018-515117 | A | 14 June 2018 |
| | | | | US | 2018-0139993 | A1 | 24 May 2018 |
| | | | | WO | 2016-185133 | A1 | 24 November 2016 |
| KR | 10-2015-0110752 | A | 02 October 2015 | CN | 105007755 | A | 28 October 2015 |
| | | | | CN | 105007755 | B | 03 March 2020 |
| | | | | EP | 2948001 | A1 | 02 December 2015 |
| | | | | EP | 2948001 | A4 | 19 October 2016 |
| | | | | EP | 2948001 | B1 | 29 September 2021 |
| | | | | JP | 2016-510215 | A | 07 April 2016 |
| | | | | JP | 2018-143255 | A | 20 September 2018 |
| | | | | US | 10098371 | B2 | 16 October 2018 |
| | | | | US | 10264809 | B2 | 23 April 2019 |
| | | | | US | 2014-0212570 | A1 | 31 July 2014 |
| | | | | US | 2014-0234479 | A1 | 21 August 2014 |
| | | | | US | 2019-0297928 | A1 | 03 October 2019 |
| | | | | WO | 2014-117163 | A1 | 31 July 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102026681

- WO 2010120923 A

**Non-patent literature cited in the description**

- **CHIN-LIN HSU et al.** *Food Chemistry*, 2003, vol. 83, 85-92